# EUROPEAN PATENT APPLICATION

(11) **EP 4 697 286 A1**
(43) Date of publication of application: **18.02.2026**
(21) Application number: 25196176.9
(22) Date of filing: 15.08.2025
(51) Int. Cl.: G06V 10/82, G06V 20/52, G06V 40/10, G06V 40/20, A01K 29/00, A61B 5/11, G06T 7/00, G06T 7/11, G06T 7/20

(54) **METHOD OF MONITORING A LOCOMOTOR SYSTEM OF AN ANIMAL USING IMAGE PROCESSING IN A MOTION ANALYSIS SYSTEM**

(30) Priority: 15.08.2024 NL 2038442
(71) Applicant: Nedap N.V., 7141 DC Groenlo (NL)
(72) Inventor: MULLER, Roxie Sabri Romero, 7141 DC Groenlo (NL); HARBERS, Arnoldus Gerardus Franciscus, 7141 DC Groenlo (NL); KRABBENBORG, Glen Peter, 7141 DC Groenlo (NL)
(74) Representative: V.O.

(57) **Abstract**

The invention is directed at a method of detecting an abnormality in a locomotor system of an animal by means of motion analysis, using a motion analysis system comprising a camera configured for monitoring at least a part of a pathway wherein the animal travels, wherein the camera is operatively connected to a computing device and a data storage of the analysis system, and wherein the computing device comprises a processor and wherein the computing device is communicatively connected to the data storage. The invention is further directed at a motion analysis system.

## Description

### Field of the invention

The present invention is directed at a method of monitoring a locomotor system of an animal by means of motion analysis, using image processing in a motion analysis system, the motion analysis system comprising a camera configured for monitoring at least a part of a pathway wherein the animal travels, wherein the camera is operatively connected to a computing device and a data storage of the analysis system, and wherein the computing device comprises a processor and wherein the computing device is communicatively connected to the data storage. The invention is further directed at a motion analysis system.

### Background

Although the present document includes references to various other documents, no admission is made that any reference constitutes prior art. The discussion of references refers to their content as presented therein, and does not acknowledge nor confirm the accuracy or pertinency thereof. It will be understood that, although a number of prior art publications may be referred to herein, this reference does not constitute an admission that any of these documents form part of the common general knowledge in the art in any country.

Lameness in cows is a well-known issue on farms, with an average prevalence of 20%-25%. Detection of lameness is based on visual observations. This method is subjective and labor-intensive. Often, lameness detection is an additional task for employees, which leads to late detection of lameness cases, thereby increasing the damage in terms of health and production loss more than necessary. Lameness is not only a problem encountered with cows, but occurs in various other farm animals as well, including horses, sheep, goats, and pigs. It may result from a range of causes, such as injury, infection or bone problems. Addressing lameness requires a combination of good animal husbandry, early detection, and prompt treatment.

In terms of detection, various lameness detection systems and methods based on image processing are available on the market. These systems apply image recognition in different ways, but are typically directed at the detection and tracking of body features from which a locomotor score may be calculated. In addition, various different systems and methods enable detection lameness in cattle, horses, pigs or other animals based on three dimensional video of animals from different fields of view (e.g. from aside or above), wherein the video is analyzed using artificial intelligence. The available systems often suffer from being insufficiently accurate in the detection of lameness or other defects in the locomotor system for a variety of reasons. In the best case, this could lead to a relatively large number of false positives, i.e. animals that are separated with presumed locomotor defects which - after inspection - turn out to be free of such defects. It may however also lead to false negatives, for example in those cases where a locomotor defect is not recognized by the system while actually being present. This may for example occur in case a system is unable to discriminate between two animals both being visible in the video at the same time, and therefore discards the image sequence or provides an incorrect result. Due to the lesser amount of data obtained from a two dimensional video, the above issues are even worse for systems that rely on the processing of two dimensional image sequences.

### Summary of the invention

It is an object of the present invention to provide a method of monitoring a locomotor system of an animal by means of motion analysis, using image processing in a motion analysis system, that leads to a reliable outcome in the determination of a potential occurrence of the abnormality in the locomotor system in the animal, to enable an operator to take action in order to allow examination of the animal to perform a health check. The invention is directed at providing a method for a decision support system or action system that enables to target the right animals for such a health check, and by no means intends to provide a diagnostics method itself. The present invention additionally intends to provide a motion analysis system.

To this end, there is provided herewith a method as described above, wherein the method comprising the following steps. The processor receives, from the camera, a sequence of images of the animal moving along the pathway the sequence of images forming a video of the moving animal, wherein each image of the sequence of images is associated with a time stamp comprising timing data indicative of a moment of capturing the image by the camera. The processor detects in a plurality of the images of the sequence, the animal by recognizing in each image of the plurality of images a body of the animal. Further, the processor selects a subset of consecutive images from the sequence of images such as to form a video clip comprising the subset of consecutive images, wherein the video clip is representative of a fragment of the video. The processor further determines, using the time stamps of two or more images from the subset of consecutive images, a velocity of the animal. The processor also classifies, using a machine learning data processing model, a motion of the animal from the video clip as being indicative of an abnormality in the locomotor system, and provides an outcome of said step of classifying as classification data at an output based on the output of the machine learning data processing model. Furthermore, the processor provides as an outcome of the method, an output signal dependent on the classification data being indicative of an occurrence of the abnormality in the locomotor system in the animal, wherein the output signal is further dependent on the velocity of the animal.

In accordance with the present invention, the velocity of the animal - a typical velocity associated with the respective animal(s) in the video clip - is used as an additional classifier in order to improve the reliability of the outcome. In particular, the invention is based on the insight that in order to improve reliability of an evaluation of any potential locomotor defect, those parts of a video indicative of a relatively high walking velocity of the animal are more important than parts of a video associated with a relatively low walking velocity. This is based on the insight that low walking speed does often not reflect a true locomotor system status expression, because the movements made by an animal at low velocity are often influenced or triggered by events or motivations other than a desire of the animal to travel to a different location. It is in particular the low velocity parts that give rise to noise in the determination process. For example, where multiple animals are visible in the same image sequence this is often caused by at least one of the animals standing still or having a low velocity. But even if the system is able to distinguish between the animals in the image, low velocity is often caused by numerous other factors, which likewise influence the way an animal moves. For example, an animal walking at a certain walking speed may suddenly slow down because of being distracted or due to sudden stress factors, fear or hesitation, or simply not being in the spirit to move further or being tired. These emotions or effects result in various parts of the body moving differently, because the animal as a whole is acting differently at that moment. The present method takes the velocity of the animal in the video clip into account, and thereby is able to select/discard/weigh/adapt/modify the classification data in order to improve the reliability of the outcome.

In accordance with the method, a single animal in the pathway may be monitored, so that determining the velocity enables it to associate the velocity with the monitored animal in the pathway. The velocity may also be associated with the video clip, e.g. where the monitored animal is identified at a later stage (e.g. by an animal management system or by a user). If the identity of the animal is known or can be identified during the method, the velocity determined may be associated with the identified animal directly. Because the velocity is determined for the (or each) video clip and may differ from video clip to video clip if there are multiple clips, the velocity is likewise associated with the respective video clip. If multiple animals are monitored in the pathway simultaneously, e.g. by being in the image sequence at the same time, each of the animals to be monitored will be associated with its own velocity. In this case, video clips may be formed for each animal in the video, e.g. by truncating the video at its ends (at the image(s) wherein the head/tail of each animal enters/leaves the image in the sequence, for example), and by dividing the truncated video in parts suitably to form the video clips for each animal. As may be appreciated, in that case (some of) the video clips may still include multiple animals in the images of the respective clip, but the method of the present invention in various embodiments is able to deal with that, as will be explained later. For example, in more basic variants of the invention, contours of animals that are not the subject of evaluation may be masked, but yet in other implementations of the invention masking is not necessary. For example, if contours from different animals in the sequence can be identified and distinguished, these may also be tracked individually. This would make the need for masking obsolete. Furthermore, if a video vision transformer is applied as the machine learning data processing model for classifying, the model is able to distinguish and follow the different animals due to the use of attention modules in the encoder and decoder layers.

As stated, the method of the present invention applies a machine learning data processing model to classify a motion of the animal from the video clip as being indicative of the abnormality in the locomotor system, and provides an outcome of said classification as classification data at an output based on the output of the machine learning data processing model. What is meant thereby is that the video clip, formed of the images that include the body of the animal, is provided as input to the machine learning data processing model, which is trained to recognize body features and their locations in the images of the video clip and evaluate the body motion throughout the video clip (in the basis of the motion of these features) as being indicative of an abnormality in the locomotor system. With features, it is meant that these could include recognizable body parts or shapes (such as joints, legs, feet, bones, etc.) or the overall shape of the contour of the animal (which is preferably be taken from above with a camera). The abnormality could be a specific abnormality searched for or an abnormality from a plurality of different known abnormalities that may include. As may be appreciated, also the absence of an abnormality may be the result of the classification, i.e. the animal walking normally with no or no significant deviations from a normal walking motion.

In some embodiments, the step of classifying comprises determining, by the processor using the machine learning data processing model, whether or not the abnormality in the locomotor system occurs, wherein the classification data is a Boolean classifier indicative of the outcome of the classification. This could be an indication of a specific locomotor defect occurring or not, for example. In yet further embodiments, the step of classifying comprises determining by the processor using a machine learning data processing model, a probability value indicative of a probability of the abnormality in the locomotor system occurring in the animal. This is similar to the Boolean classifier, but providing more information on how probable it is that the defect is indeed occurring.

In yet further embodiments, the step of classifying comprises determining by the processor using the machine learning data processing model, a classifier value indicative of an outcome within a range of outcomes, such as a class of abnormality or of a range of probabilities. In these embodiments, the outcome may be a class identifier that indicates, e.g. severeness of a certain locomotor defect, or a name of a specific locomotor defect selected from a variety of defects for which the machine learning data processing model has been trained, or a class identifier indicative of a probability sub-range. Any desired class identifier may be applied here.

In some embodiments, for implementing the velocity dependency of the outcome, the step of providing the output signal comprises a step of evaluating the classification data based on the velocity of the animal. For example, the outcome may be modified in order to take the velocity of the animal into account. In other embodiments, the velocity may be used in order to select certain video clips or parts of a video over other video clips or parts of a video, or alternatively to discard specific parts wherein the velocity of the animal concerned is low. Yet other embodiments use the velocity of the animal in order to weigh the classification data and provide a corrected outcome based on such weighing, for example either directly or using a normalized velocity correction factor (e.g. *υ* / *υₘₐₓ,* where *υ* is the velocity of the animal in the specific video clip concerned and *υₘₐₓ* is the maximum velocity in the video) or another weighing factor taking the velocity into account as desired. As may be appreciated, this may be a weighing factor that is linearly dependent on the velocity, or it may be otherwise correlated therewith in a desired way, such as positively correlated, non-linearly correlated, or correlated such as to promote a certain range of velocities over others. The below will provide additional manners in which this may be implemented.

In some embodiments of the above, the step of evaluating comprises selecting or discarding the classification data based on the velocity of the animal being above or below a predetermined threshold, wherein the classification data is selected if the velocity is above the threshold, and wherein the classification data is discarded if the velocity is below the threshold. This may be helpful in order to very easily base the outcome of the method only on classifier data associated with sufficiently large animal velocity, i.e. above the predefined or pre-set threshold.

In some other embodiments, the classification data comprises a probability value indicative of a probability of the abnormality in the locomotor system occurring in the animal, scaling the probability value based on the velocity, by weighing or multiplying the probability value with a weighing value which is dependent on the velocity. In this case, any probability value that is indicative of a large probability of a locomotor defect will be diminished in value if the velocity is lower. This correction is dependent on the velocity determined.

In some embodiments, the method further comprises the steps of receiving, by the processor from a radio frequency identification transceiver, an identification signal comprising identification data; and associating the identification data with the animal. This likewise directly enables to associate any desired data from the method with the animal identified, in order to store this in a data repository that may be accessible with an animal management system. As may be appreciated, the application of a radio frequency identification (RFID) transceiver that enables to receive the identifier data stored in animal identification tags is optional. Although this feature provides significant advantages in order to directly associate the locomotor system data with the correct animal and store this in an animal management system, as an alternative, it is also possible to implement the method advantageously without this feature. For example, a farmer operating the system may manually provide the identification data in a less advanced but cost effective implementation, or the method may be accompanied with an image recognition feature that allows to recognize and identify each animal without using an RFID transceiver. The method may also be implemented with both an RFID transceiver and such an image recognition feature as a complementary part to improve identification if multiple RFID signals are received without providing a certain identification of a specific animal.

In yet further embodiments of the method, the step of selecting, by the processor, a subset of consecutive images from the sequence of images comprises selecting, by the processor from the sequence of images, a plurality of subsets of consecutive images such as to form a plurality of video clips, each video clip of the plurality of video clips comprising a unique one of the subsets of consecutive images, wherein each video clip is representative of a unique fragment of the video, wherein optionally the fragments of two or more video clips at least partially overlap in time. In these embodiments, the walking path of an animal is divided in several video clips, and for each video clip an animal walking speeds is determined. This animal walking speed may for example be used in order to select the video clips that are most suitable for determining whether or not an abnormality in the locomotor system is present and/or which abnormality. This selection possibility allows to greatly improve the quality of the determination or detect faulty passings.

In some of these embodiments, the step of selecting the plurality of subsets of consecutive images to form the plurality of video clips comprises applying a sliding window of a subset of consecutive images to the video. For applying the sliding window, the step of selecting may optionally comprise a number of sub-steps. For example, these sub-steps could include: selecting, for each image of the sequence of images, a fixed number of consecutive images that precede the image concerned, wherein the fixed number determines a duration of the sliding window. The sub-steps could further include: forming a set of images by arranging the consecutive images including the concerned image in sequential order in time, the set of images forming a momentary video clip of the sliding window, wherein the momentary video clip is associated with the image concerned. Furthermore, the sub-steps could include: associating the momentary video clip with the time stamp associated with the image concerned; and further these sub-steps could include: forming the sliding window by providing each momentary video clip with the respective time stamp associated therewith, such as to yield the plurality of video clips. From the sliding window, for example, the most suitable video clip(s) could be selected based on the velocity, in order to obtain a reliable determination of an abnormality in the locomotor system. There are multiple ways in which this may be applied in order to improve the reliability.

In some embodiments, the steps of classifying the motion of the animal and determining the velocity of the animal are performed for each video clip of the plurality of video clips for obtaining the classification data and velocity for each video clip, wherein the method further comprises determining, for each video clip and based on the velocity associated with the video clip, a weighing factor, wherein the weighing factor is dependent on and positively correlated with the velocity, and associating the weighing factor with the classification data associated with the video clip for indicating a significance thereof for each video clip. In determining, for providing the output signal dependent on the classification data being indicative of the abnormality in the locomotor system in the animal, the output signal may for example be dependent on the classification data and the associated weighing factors of the plurality of video clips. The weighing factors may be determined in various different manners. For example, in some embodiments, the weighing factor, for each video clip, is linearly dependent on the velocity. Alternatively or additionally, the weighing factor, for each video clip, may be clipped to either one of zero or unity (or another suitable value) dependent on whether the velocity is respectively below or above a threshold. Also, the weighing factor, for each video clip, may be non-linearly dependent on the velocity. For example, for some preferred velocity ranges the weighing factor may indicate this velocity to be much more relevant for determining an abnormality in the locomotor system.

In the more common embodiments, the machine learning data processing model has been trained to provide at its output a probability value indicative of a probability of the abnormality in the locomotor system occurring in the animal based on receiving at its input the video clip or video clips as main input (or sometimes as only input). In these embodiments, the machine learning data processing module applied in accordance with the present novel and non-obvious concepts, is not and does not need to be trained on the basis of the velocities of animals in video clips. In this case, a supervised training method for example may include feeding video clips of animals with or without an occurring abnormality in the locomotor system to the input of the model, and using data on whether or not an abnormality is occurring (and optionally which type of abnormality) as ground truth data to perform the training. Unsupervised training may for example initially be based on feeding arbitrary video clips of walking animals (thus without preselecting the video clips used as input), and to leave the model to find out those video clips that are out of the ordinary (e.g. based on statistics) in order to perform training during a pre-training step. The fine-tuning may then be supervised, as described. The training, in preferred basic but robust (i.e. very reliable) embodiments, may also include feeding only video clips with animals showing a normal walking pattern, in absence of any abnormality. Preferably, only video clips are fed wherein the walking velocity of the animals is relatively normal. With the walking velocity being 'relatively normal', it is meant here that the velocity of the animal is not too low but also not too high. This velocity may thus be above a first threshold and below a second threshold, for example where the first and second threshold may be provided by the average or median walking velocity of all animals of a population plus or minus a multiple (e.g. one or two) times the standard deviation. Other criteria may likewise be applied to provide a good range for this. The model will then learn to recognize video clips that deviate from a normal walking pattern, and these are thus suspect video clips of animals wherein an abnormality in the locomotor system occurs, or which should at least be seen for inspection and thus are to be separated from the group.

However, other embodiments may take all this a step further. In some embodiments, the machine learning data processing model has been trained to provide at its output a probability value indicative of a probability of the abnormality in the locomotor system occurring in the animal based on receiving at its input said video clip and said velocity of the animal associated with the video clip. The classification data provided at the output of the machine learning data processing module thereby is provided including the dependency on the velocity of the animal, enabling to provide the output signal directly based on the classification data. The machine learning data processing model will optimize the training result including the velocity parameter, and will thus achieve the best matching result taking the velocity or a weighing factor into account. As a result, the video clips relating to indefinite animal behavior at low walking velocities or stand still, will automatically be discarded or sufficiently demoted in the classification data to prevent these disturbing the end result.

In other or further embodiments or the method of the present invention, the output signal comprises at least one of: an indication of a grade of severeness of the abnormality in the locomotor system occurring in the animal, an indication of whether or not a certain abnormality is present, a probability value or range classifier indicating an estimate of the probability value of an abnormality (or a specific type of abnormality) being present, or a type of abnormality in the locomotor system occurring in the animal.

In some embodiments, the method further comprises a step of controlling, by the processor dependent on the output signal, a separation gate for enabling separation of the at least one animal for further examination, such as a health check. Alternatively, it is also possible to provide the output signal to a display device, a mobile phone or a laptop, in order to present the information to an operator. As may be appreciated, another possibility is to store, based on the output data, an outcome of the method as animal management data associated with the animal in an animal management system.

In yet further embodiments, the step of detecting, by the processor in a plurality of the images of the sequence, the animal, comprises a step of segmenting the images such as to recognize a body contour of the animal. Alternatively, bounding boxes may be used in order to identify the whereabouts of an animal in an image, and in order to determine its speed. To enable detection of abnormalities in the locomotor system, the use of bounding boxes is insufficient because they do not provide information on the movement of various body parts in relation to each other. However, this information may be obtained from the full images depicting the body of the animal. Using contour recognition, the body contours may be obtained from each image, which provide already more information on the potential presence of a defect in the locomotor system. However, a particular advantage is the fact that using contour recognition, it becomes also possible to distinguish between multiple animals in the image, even if the body contours are overlapping. This may be the case, for example, if two animals are standing or moving contiguous to each other, for example as with a cow that tries to force itself past another cow, or a calf closely following its mother and being partly hidden from the view. Because the contour of an animal from above is to some extend predictable, depending on body size and weight, it is possible to develop an algorithm or train a machine learning model to distinguish between two body contours that blend together in an image. Furthermore, apart from the fact that the predictability enables to separate the body contours in such cases, typically in most video clips the body contours are not overlapping in all of the images of the clip, such that there are always several images wherein the two contours are not overlapping. The contours of two animals may thus be determined or learned from those images, enabling to distinguish these in images wherein they are overlapping.

In some of the abovementioned embodiments, the step of segmenting is therefore performed by an image recognition module comprising an image recognition machine learning data processing model, wherein the image recognition machine learning data processing model has been trained to recognize a contour of an individual animal. Training of the image recognition machine learning data processing model may be achieved relatively straightforward using supervised learning, by providing training images of contours of the type of animal to be monitored later, and provide the contour data associated with each training image as reference data in order to perform the training. To improve system performance and prevent long training periods upon installation of a system, it is possible to apply a transfer learning method wherein the system is built on the basis of a pre-trained model with generic training data of, for examples, cow body contour data from an top-down camera position. After installation of the system on-site, only a limited amount of post-training is required in order to adapt the system to the cow and surroundings on-site. This post-training is not required, and where desired it can be done while the system is already fully operational. For example, the system may be implemented and be made operational, and thereafter improve in use while processing each new video clip.

In some of the above embodiments, the image recognition machine learning data processing model has been further trained to separate two or more contours of individual animals (i.e. the number of contours being equal to the number of animals), in a situation wherein the two individual animals are contiguous to each other such that their body contours blend together in the image. This has already been briefly explained above. Once the system is able to recognize the different body contours of various animals, it may further be trained to continue recognizing different body contours in a single image, also where these contours may be (partly) overlapping. Although this may already be achieved using, for example, a convolutional neural network (CNN) as image recognition machine learning data processing model, it may work also very well or even better using a machine learning data processing model that is able to carry over data from previous image evaluations to evaluate images with overlapping body contours. Therefore, instead of or in addition to the application of a convolutional neural network as image recognition machine learning data processing model, the image recognition machine learning data processing model may also be implemented using a recurrent neural network (RNN), a long short-term memory (LSTM), a vision transformer (ViT) or a video vision transformer (ViViT). Regarding the latter, it is observed that a video vision transformer architecture includes both a spatial transformer and a temporal transformer that cooperate with each other in order to allows for parallel processing of video frames. The spatial transformer thereby applies a CNN structure to extract features from the images, which are weighed using the attention mechanism of the spatial transformer model. In a similar manner, the temporal relations between features of different images are processed by the temporal transformer, using a similar or same attention mechanism to weigh the importance of different frames in the video data.

In some embodiments of the method of the present invention, the camera is positioned above the pathway, such as to obtain image of the animal from above. It has been found that a particular well working implementation of the present invention is provided based on one or more 3D cameras that observe the at least one animal from above, from a top-down viewpoint. The animal is then imaged on its back. The motion of the spine during walking, but also any head and neck motion, deviations in motion and body positions during walking, as well as typical motion patterns of legs, shoulders or hip, are well visible from above and are good indicators of potential lameness. The term '3D camera' used herein must be broadly interpreted to indicate any camera, vision registration device, system of cameras or system of vision registration devices that enable to obtain image data in three dimensions. This includes real 3D cameras, but likewise certainly also RGB camera's recording 2D images of a body contour and using coloring or texturing of the images to provide height data.

In yet further embodiments, the step of detecting the animal further comprises a step of body feature recognition of the body of the animal, for recognizing at least one of a hip, a spine, a shoulder, a leg, a neck or a head of the animal. As already described above, motion of the spine during walking, but also any head and neck motion, deviations in motion and body positions during walking, as well as typical motion patterns of legs, shoulders or hip, are well visible from above and are good indicators of potential lameness.

In some embodiments, the machine learning data processing model, or the further machine learning data processing model is at least one of a group comprising: a neural network, such as a hierarchical neural network, a convolutional neural network, a convolutional-deconvolutional neural network, such as a U-net type neural network, a random forest model, a recurrent neural network, a long short-term memory, a vision transformer or a video vision transformer. Hereinbefore, mention has also been made of an image recognition machine learning data processing model for contour recognition, in particular also for separating two contours of two individual animals, in a situation wherein the two individual animals are contiguous to each other such that their body contours blend together in the image. This image recognition machine learning data processing model as well as the machine learning data processing model and the further machine learning data processing model may be provided by separate machine learning models or may be integrated in one or more machine learning data processing models. These could include one or more of the machine learning data processing model architectures referred to above.

In some embodiments, the pathway is a lane or passage wherein the animal is enabled to move through. Yet in some other embodiments, the pathway is provided by an area wherein the animal is enabled to move freely in multiple directions or a myriad of directions, the camera being configured to monitor the area, and wherein the method further comprises obtaining, from the each image of the subset of images of the video clip, an orientation of the animal and a position of the animal in the area, and segmenting the images of the video clip such as to correct a current orientation of the animal to a reference orientation of the animal, the reference orientation being predetermined, and wherein the step of determining the velocity of the animal further comprises determining the velocity along a trajectory following the positions of the animal obtained from the images of the subset. In scenarios where animals do not walk in a straight line, the accuracy of velocity determination could be compromised. To address this issue, in the latter case above, a technique is employed, in accordance with some embodiments, that ensures the animal is always centrally positioned within the image frame through a process of segmentation. This technique normalizes the route taken by the animal, allowing the motion analysis system to accommodate multiple possible walking paths. As a result, the system is able to more accurately estimate the animal's velocity. As may be appreciated, by normalizing the animal's route, the system can accurately determine the velocity of the animal, regardless of variations in its walking path. This enhancement ensures that velocity calculation is consistent and reliable. The technique further allows the method and system to handle various walking routes, which versatility means that the system can accommodate deviations from a straight line, which are common in various environments.

In accordance with a second aspect of the present invention, there is provided a motion analysis system for detecting an abnormality in a locomotor system of an animal by means of motion analysis. The system includes a camera, wherein the camera is configured for monitoring at least a part of a pathway wherein the animal travels. The system also includes a computing device and a data storage communicatively connected to the computing device. The camera is operatively connected to the computing device and the data storage. The computing device comprises a processor, and this processor is configured for controlling the system and for processing instructions which, when carried out, operate the system such as to perform a method in accordance with the first aspect described above. In particular, this method comprises the steps of: receiving, by the processor from the camera, a sequence of images of the animal moving along the pathway the sequence of images forming a video of the moving animal, wherein each image of the sequence of images is associated with a time stamp comprising timing data indicative of a moment of capturing the image by the camera; detecting, by the processor in a plurality of the images of the sequence, the animal by recognizing in each image of the plurality of images a body of the animal; selecting, by the processor, a subset of consecutive images from the sequence of images such as to form a video clip comprising the subset of consecutive images, wherein the video clip is representative of a fragment of the video; providing, by the processor, the video clip as input to a machine learning data processing model, wherein the machine learning data processing model is trained to classify a motion of the animal from the video clip as being indicative of the abnormality in the locomotor system, and providing at an output of the machine learning data processing model an outcome of said classification as classification data; determine, by the processor, using the time stamps of two or more images from the subset of consecutive images, a velocity of the animal; and providing, by the processor as an outcome of the method, an output signal indicative of an occurrence of the abnormality in the locomotor system in the animal, wherein the output signal is based on the classification data and on the velocity of the animal.

As may be appreciated, the various entities of the system may be implemented as a dedicated system operative on a farm, a zoo, a petting zoo, or any other facility for housing animals or groups of animals. It may also be implemented as a distributed system, wherein one or more entities of the system are interconnected by wireless or wireline connections to a data communication network. This data communication network could be a local area network or a wide area network for example, and it is not per se required that the computer system and the camera or any other entity of the system (save for the camera and the optional RFID reader, which are operative in each others vicinity) are located in each others environment. For example, one or more of the computing device, the processor and the data storage may be interconnected to any of the other parts of the system via a data communication network. Also, multiple systems in accordance with one or more embodiments may access centralized parts of the system via a server on a wide area network, for example such as to provide the system as a cloud based implementation. The data storage itself may likewise be distributed amongst various locations, for example to include a local data storage for storing video data locally and to access the machine learning data processing model or the further machine learning data processing model as a cloud based service. Naturally, the video data (or parts thereof) may also be stored in a cloud storage device. The skilled person will recognize the various optional implementations with respect to local or decentralized parts of the system accessible via one or more data communications networks.

In some embodiments, the system of the second aspect further comprises one or more radio frequency identification reader stations for communicating with one or more radio frequency identification transceivers. Here the processor may be further configured for processing instructions that enable the system to perform the steps of: receiving, by the processor from at least one of the radio frequency identification transceivers, an identification signal comprising identification data; and associating, by the processor, the identification data with the animal. In other or further embodiments, the camera is positioned above the pathway, such as to obtain image of the animal from above.

### Brief description of the drawings

The invention will further be elucidated by description of some specific embodiments thereof, making reference to the attached drawings. The detailed description provides examples of possible implementations of the invention, but is not to be regarded as describing the only embodiments falling under the scope. The scope of the invention is defined in the claims, and the description is to be regarded as illustrative without being restrictive on the invention. In the drawings:
Figure 1 schematically illustrates a motion analysis system in accordance with an embodiment of the invention, for carrying out a method in accordance with an embodiment of the invention;
Figure 2 schematically illustrates an exemplary evaluation carried out using a method in accordance with an embodiment of the invention;
Figure 3 schematically illustrates an exemplary evaluation carried out using a method in accordance with an embodiment of the invention;
Figure 4 schematically illustrates an exemplary evaluation carried out using a method in accordance with an embodiment of the invention;
Figures 5A to 5C schematically illustrates an example of a weighing applied using a method in accordance with an embodiment of the invention;
Figure 6 schematically illustrates contour recognition using a machine learning data processing model, in an implementation of the invention, in accordance with an embodiment;
Figures 7A and 7B schematically illustrate contour recognition and distinguishing of several animals in overlapping contours, using an implementation of the invention in accordance with an embodiment;
Figure 8 schematically illustrates a method in accordance with an embodiment of the invention;
Figure 9 schematically illustrates a method in accordance with an embodiment of the invention;
Figure 10 schematically illustrates contour recognition using a machine learning data processing model, in an implementation of the invention, in accordance with an embodiment;
Figure 11 schematically illustrates a training method for training any of the described machine learning models for carrying out any one or more of the method steps of the method in accordance with an embodiment of the invention.

### Detailed description

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

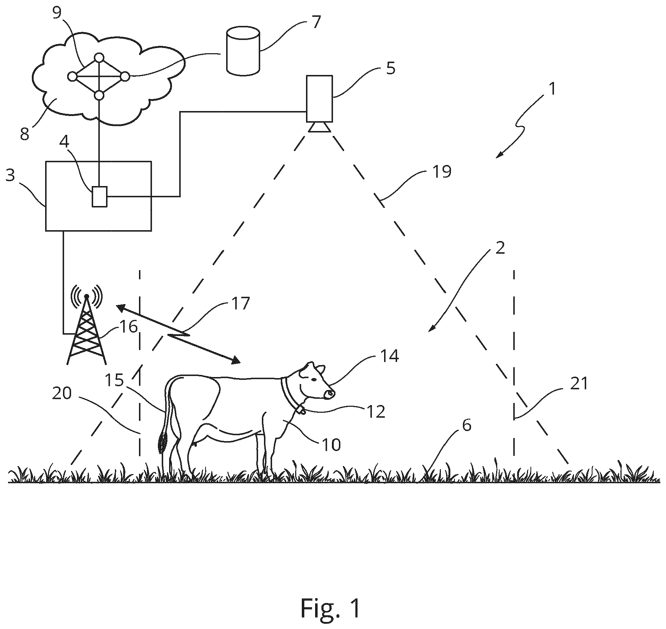

Figure 1 schematically illustrates a motion analysis system 1 in accordance with an embodiment of the invention, for carrying out a method in accordance with an embodiment of the invention. In the system 1, a camera 5 takes video recordings of a pathway 2 with floor 6 wherein animals 10 may be progressing their way. The camera 5 is mounted above the pathway 2, providing a top-down view of the pathway (or at least a part thereof). The camera 5 is a three dimensional or 3D camera and is thereby suitable for taking a three dimensional video. As may be appreciated, the camera 5 may include any camera or sensor device, or a combination or system of devices, suitable for obtaining the 3D video recordings. For example, the camera 5 could be implemented by a system of one or more cameras enabling to take stereo images. Another option would be a single camera 5 taking a 2D image from above, which is augmented with height data by means of a height dependent coloring of pixels, i.e. the height data being encoded in the pixel colors in each image. This latter implementation may herein be referred to as an RGB-type camera 5 (the letters R G and B in the acronym referring to the colors red, green and blue). Each image from camera 5 thus includes 3D data of the scene that is imaged.

The dashed lines 19 indicate the field of view of the camera 5, which may optionally be limited by borders 20 and 21 indicative of registration borders 20 and 21 for the camera 5. These registration borders 20 and 21 for example trigger the registration of video data when an animal 10 moves in between the borders. For example, the processor 4 of the computing device 3 may discard any video images which depict only a part of the animal 10. The animal 10, a cow, may for example be registered once its tail 15 is between registration borders 20 and 21 and until its nose 14 (or head (no reference numeral), if desired) is still between registration borders 20 and 21. As may be appreciated, the exact manner of implementing when to start and when to cease registration, e.g. as triggered by the location of certain body features, is not prescribed here and may be selected by the skilled person, as long as it yields useable video data. The borders 20 and 21 may be implemented virtually, e.g. by instructing the processor 4 properly to determine the above. However, alternatively it is also possible to physically implement some sort of trigger means, such as an optical trigger (e.g. optical sensor with light module; or a laser with sensor) or a pressure sensor on the floor 6. The skilled person will recognize several ways of implementing this feature in some sort of manner.

The system further includes a radio frequency identification (RFID) module consisting of an RFID reader 16 configured for receiving an RFID signal 17 from the nearby cow 10. The RFID signal 17 is transmitted from an RFID label 12, which in the present example is attached to the neck of the cow 10. The label may alternatively or additionally also be implemented as an ear tag, tail tag, leg tag or another type of RFID tag, without departing from the inventive concept described herein. If multiple animals 10 are in the pathway 2, which are fully or partially within the registration borders 20 and 21, or within the field of view 19, multiple RFID signals, one for each of these cows 10, will be received by the RFID reader 16. In that case, it may be resolved e.g. using the images from the video stream of the camera 5 combined with e.g. the signal strength of each RFID signal or its time of flight data, which RFID signal 17 should be associated with which cow or animal 10. The application of an RFID module, and in fact the use of RFID data or alternative manners of identifying the animal 10, although greatly advantageous in terms of automatization, are optional to the invention and the invention may be implemented without this feature. For example, if a single cow is led through the pathway 2 which was led there on initiative by the farmer who suspected a locomotor defect, the identification of the cow 10 is not per se necessary, because the farmer knows which cow 10 is sent through the pathway 2 Furthermore, there are alternatively other ways of recognizing the cow 10 from the video images, which are not further described here.

The images forming the video stream from camera 5, as well as the (optional) RFID data from the RFID signal 17, are conveyed to a computing device 3. The computing device 3 comprises a processor 4 communicatively connected to an internal or external memory (not shown). The computing device 3 may further be operatively connected to a data storage 7. This may be a local data storage 7, but may - as illustrated - also be a data storage 7 that is connected via a data communication system 9, such as a local network or a network which may be part of e.g. a wide area network 8. Any data retrieved or stored, for example as part of an animal management system for a farm, may be stored or distributed across the internal or external memory or the data storage 7 described; or may be conveyed (e.g. via the wide area network 8) to another means, such as a mobile device or another computer (not shown). The computing device 3, for example, may access stored data comprising instructions which enable the processor 4 to carry out a method as described herein, in accordance with any embodiments thereof. Furthermore, the stored data may additionally represent one or more machine learning data processing models that enable the processor 4 to carry out evaluations, segmenting of images or contour recognition in accordance with any embodiment of the invention.

In accordance with embodiments of the invention, the motion analysis system 1 illustrated in figure 1 is suitable for carrying out a method of detecting an abnormality in a locomotor system of an animal 10, such as the cow 10 in the figure. This detection is done by means of motion analysis. The motion analysis system 1 thereby uses the camera 5, which is configured for monitoring at least a part of the pathway 2 wherein the animal 10 travels. As may be appreciated, the pathway may be much longer than illustrated in figure 1. Also, the motion analysis system 1 may be operatively connected to other units that enable to operate various entities on a farm. In the embodiments described below, for example, a separation gate 23 will be operated responsive to an evaluation by the system 1. This gate may, for example, be controlled by processor 4, although this is of course optional. The camera 5 is operatively connected to the computing device 3, which in turn is connected to the data storage 7 of the analysis system 1. The camera, optionally, may also directly be connected to the data storage 7, enabling the computing device 3 to obtain the video data from the data storage 7.

For carrying out the method, the system 1 for example is controlled as follows using the processor 4 of computing device 3. The processor 4 receives a sequence of images of the animal 10 moving along the pathway 2 from the camera 5. The sequence of images thereby forms a video of the moving animal 10. Each image of the sequence of images is associated, by the processor 4 or already by a local processor of the camera 5, with a time stamp comprising timing data indicative of a moment of capturing the image by the camera 5. To this end, a clock unit (not shown) may be present in the computing device 3, or the time stamp may be obtained from any device connected on the wide area network 8, for example. The processor 4, thereafter, detects in a plurality of the images of the sequence, the animal 10 by recognizing in each image of the plurality of images a body of the animal 10. This may for example be implemented using a contour recognition sub-process or another segmenting process, some examples of which will be described later. The processor 4 then selects a subset of consecutive images from the sequence of images in order to form a video clip. The video clip comprises the subset of consecutive images and is representative of a fragment of the video, spanning a fraction or part of the time spent by the animal 10 between the registration borders 20 and 21. Because the animal 10 is moving through the pathway 2 toward registration border 21, the video catches the whole path followed by the animal 10 between the registration borders 20 and 21. Some animals 10 may walk along the pathway 2 in a continuous motion at an almost steady velocity, but other animals 10 may hesitate or may be distracted in another way such that their pace is not fluent and their velocity highly variable. Some animals 10 may be injured or in another way be defected in their locomotor system, and walking at a constant speed or at a normal velocity may be painful or may be impossible for these animals.

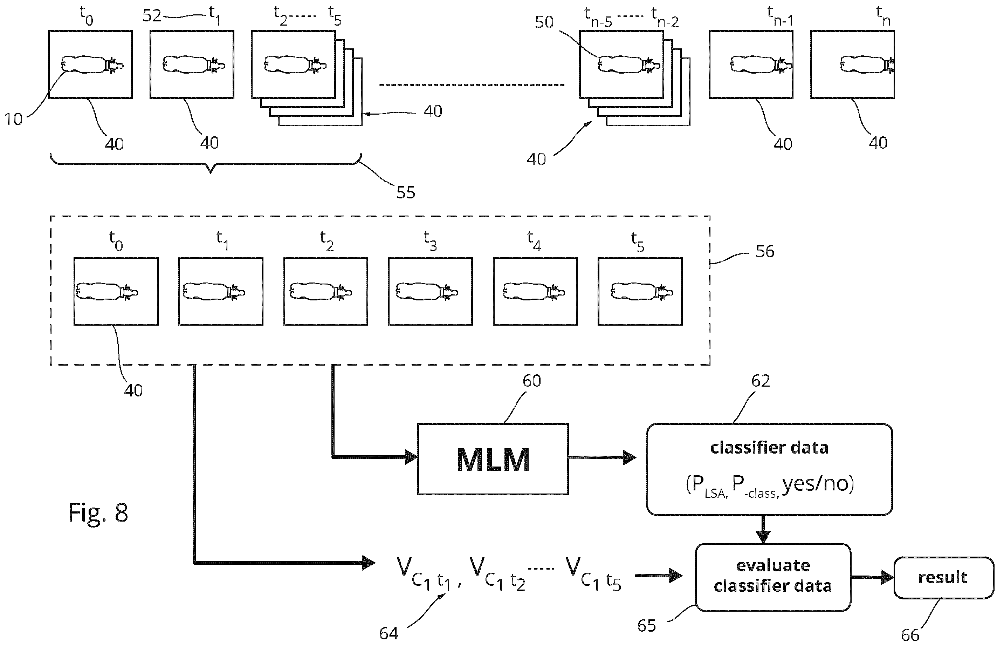

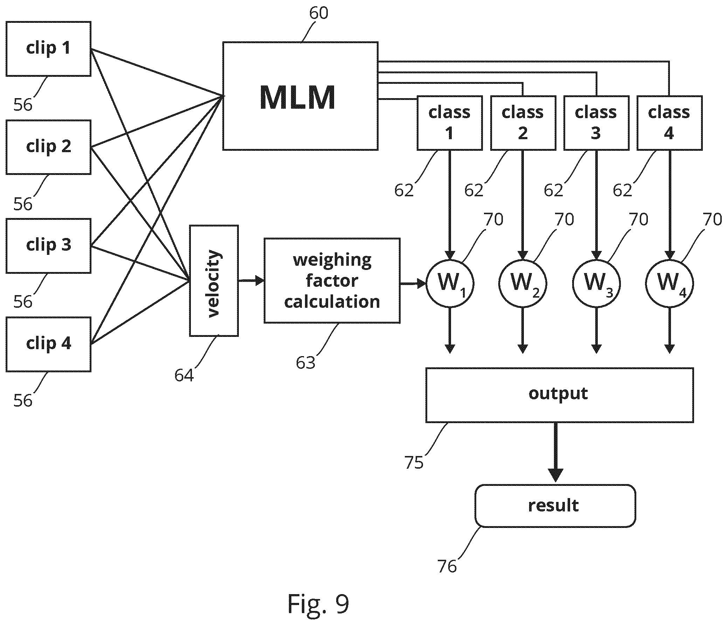

The processor 4, in accordance with the method of the invention, evaluates the motion of the animal. To do so, the processor 4 uses a machine learning data processing model 60 (e.g. see figures 8 or 9) to classify the motion of the animal 10 from the video clip. Using the machine learning data processing model 60 the motion may be classified as being healthy or normal, or as being indicative of an abnormality in the locomotor system. To give an example of an abnormality, it may be determined from the motion of the animal 10 that its left foreleg is injured, or the motion pattern found may be indicative of a disease such as an infection with bovine spongiform encephalopathy (BSE) or foot-and-mouth disease, or some other defect, such as a torn muscle or even old age. The processor 4, by applying the machine learning data processing model 60 to the images of the video clip, provides an outcome of the classification as classification data at an output. In addition to the above, the processor 4 also determines a velocity of the animal 10 using the time stamps of two or more images from the subset of consecutive images forming the video clip. This velocity is used in order to further evaluate the determined classification.

This velocity based evaluation may be implemented in various different manners. For example, the processor 4 may discard any parts of the video, or any video clips, that are indicative of a low velocity of the animal. For example, the classification data may be selected if the velocity is above a threshold, whereas the classification data may be discarded if the velocity is below this threshold. The reason for this is that a temporary low velocity of the animal 10 may be for an unknown reason that is not related to a problem in the locomotor system. Also, the velocity may alternatively or additionally be used in order to promote some parts of the video or some video clips as being more relevant for the classification than other parts of the video. For example, some abnormalities may be prominently visible at certain velocities or above some threshold velocity. In another implementation, the classification data may include probability value indicative of a probability of the abnormality in the locomotor system occurring in the animal 10, and this probability value may be scaled based on the velocity by weighing or multiplying the probability value with a weighing value which is dependent on the velocity. The processor 4 therefore, to provide an outcome of the method, provides an output signal indicative of an occurrence of the abnormality in the locomotor system (or of the motion pattern not being indicative of such an abnormality) in the animal 10, wherein the output signal is based on the classification data and on the velocity of the animal 10.
Further to the above, also the step of classifying may be implemented differently across various embodiments. This may, for example, comprise the processor 4 to determine, using the machine learning data processing model 60, whether or not a specific abnormality in the locomotor system occurs or not. In that case, the classification data may be a Boolean classifier indicative of the outcome of the classification. In yet further embodiments, the processor 4 determines, using the machine learning data processing model 60, a probability value indicative of a probability of the abnormality in the locomotor system occurring in the animal 10. In some embodiments, the processor 4 using the machine learning data processing model 60, determines a classifier value indicative of an outcome within a range of outcomes, such as a class of abnormality or of a range of probabilities. In this case, the classifier may indicate within a range of possibilities, using a score on a scale of one to five, what the odds are that a certain locomotor defect occurs with the animal 10 or whether any locomotor problem may be present.

In implementation as illustrated in figure 1, wherein an RFID reader 16 is present and RFID tags 12 are applied, the RFID data obtained from a received RFID signal 17 may directly be associated by the processor 4 with the outcome of the method, and stored as animal data in an animal management system of farm administration. The great advantage of this is that it enables the operator to monitor a complete herd automatically e.g. on a daily basis, and hence detect the occurrence of any problems at an early stage.

Furthermore, the above example is based on the selection and evaluation of a single video clip. However, advantageously it is also possible to break down a video sequence in a number of smaller fragments in order to yield a plurality of video clips. Velocities may be assigned to each clip, and the evaluation and weighing or selection may be done on the basis of these velocities.

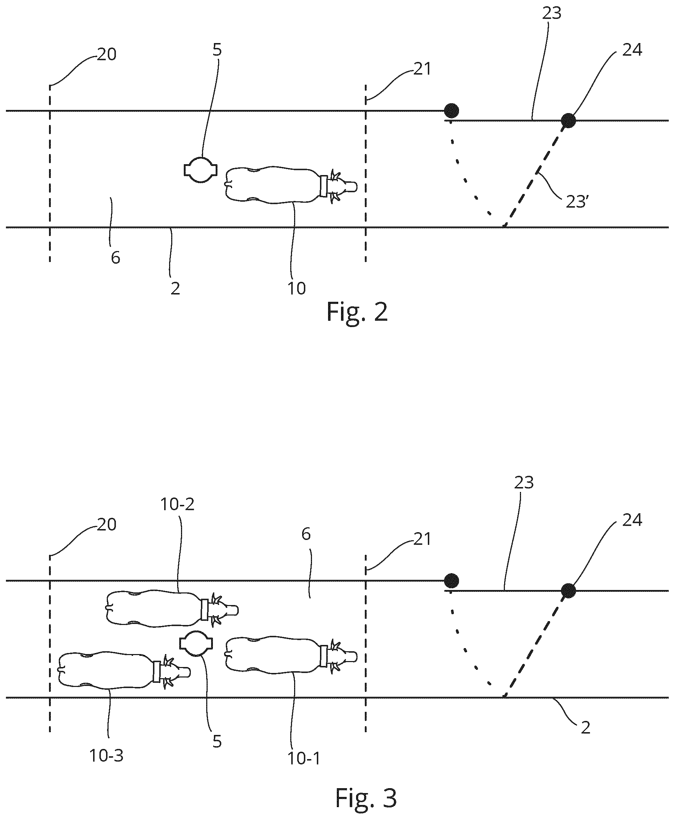

Various different situations that may occur when monitoring one or more animals 10, are illustrated schematically in figure 2, figure 3 and figure 4. In figure 2, the classic situation of a single cow in the pathway 2 is illustrated. Here, the camera 5 above the pathway 2 is positions to be aimed down at the floor 6, and thus obtains a video showing the back of animal 10. The video is being recorded and stored for further analysis as from the moment the tail 15 of the animal has past registration border 20, and the video is stopped when the animal 10 leaves the scene, i.e. when its head or nose arrives at the other registration border 21. If the evaluation performed with the motion analysis system 1 reveals that there may be a locomotor system problem (e.g. a crippled leg or foot, a wandering walk, frequent stopping, drifting or walking sideways, etc.) the processor 4 may operate a separation gate 23, which can pivot around hinge 24 using an actuator (not illustrated). The separation gate 23, in the position 23' blocks the pathway for cow 10, who will walk into a separation area where it can be examined e.g. by a veterinarian. In figure 3, it is illustrated that multiple animals 10-1, 10-2 and 10-3 may walk through the pathway 2 simultaneously, while the system is perfectly able to distinguish their contours and analyze their walk separately. The system 1 receives a sequence of images in time, and is thereby able to subsequently identify each separate contour individually, and to keep track of them. Multiple video clips for each of the animals 10-1, 10-2, and 10-3 may be taken from the source sequence obtained by the camera 5, and allow the evaluation of each animal's motion. It is also possible that the separate evaluations for each animal 10-1, 10-2, and 10-3 individually may be achieved at once, e.g. by applying a video vision transformed (ViViT) or a U-net type convolutional-deconvolutional neural network, or similar, as machine learning data processing model 60.

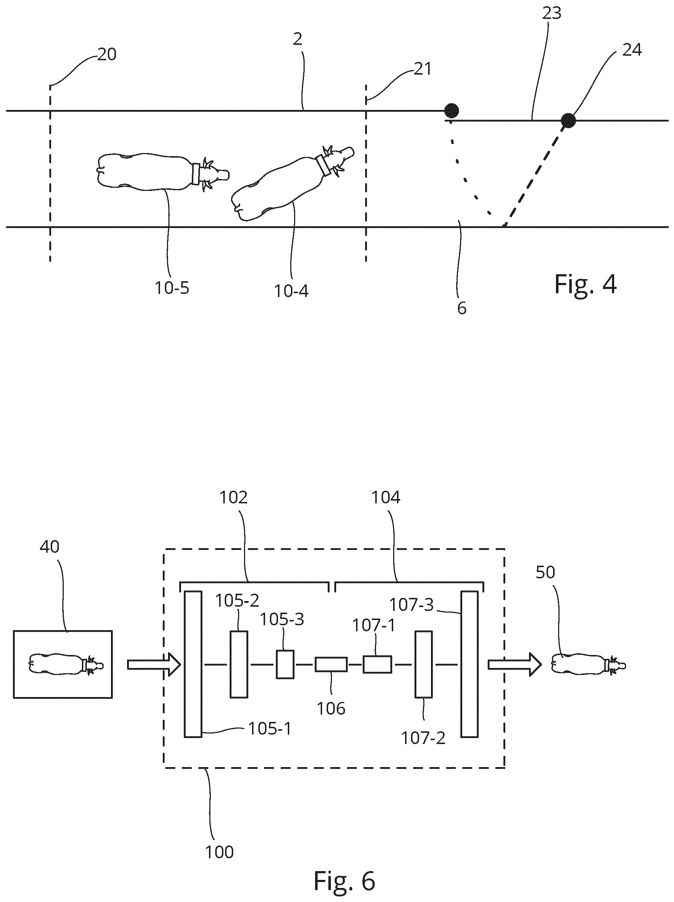

In figure 4, it is depicted that a single cow 10-4 may stand still in the pathway, blocking the path for cow 10-5. However, in the full time sequence, cow 10-4 will have arrived first and a part of its walking trajectory may have been at sufficient velocity for evaluation of locomotor system problems. Cow 10-5 arriving later still has walked the first part of its trajectory at normal walking speed, enabling evaluation as well. By discarding or demoting the low speeds parts of the video, an incorrect assessment of the locomotor system is effectively prevented.

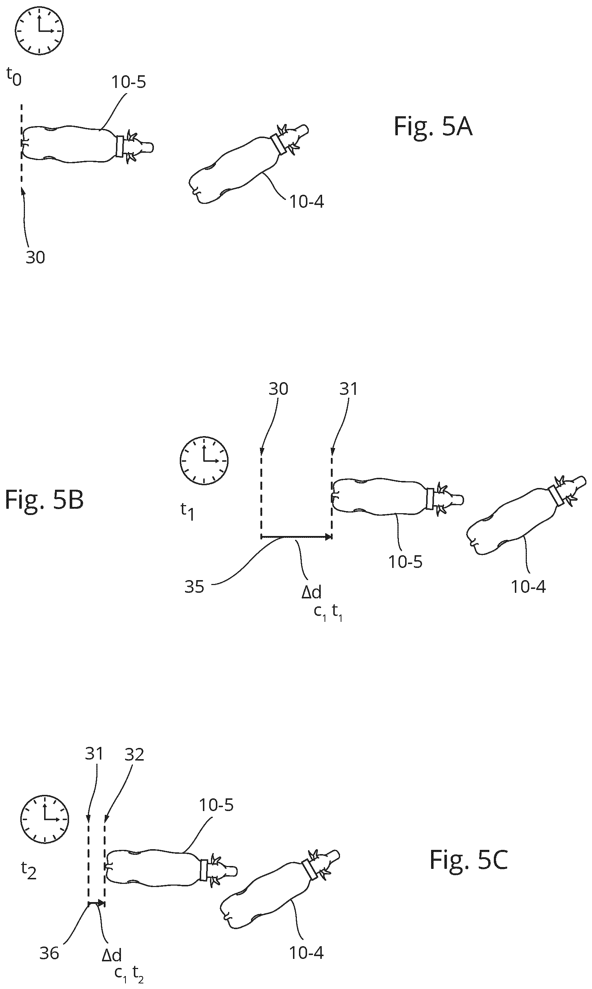

The course of the walk of cow 10-5 is illustrated in figures 5A to 5C, which also indicated the time stamps *t₀*, *t₁* and *t₂* and the distances walked Δ*d_{c1t1}* and Δ*d_{c1t2}* during each time fragment *t₀* to *t₁* and *t₁* to *t₂.* This is visualized using the dashed lines 30, 31 and 32 indicating the tail position of animal 10 at each moment in time illustrated. As may be appreciated, although the tail position is used in this example, this may likewise be any other feature of the animal body, such as the animals behind or its rear legs, front legs, head, nose, horns, etc. The distances Δ*d_{c1t1}* and Δ*d_{c1t2}* are referenced by reference numerals 35 and 36 respectively. From this, momentary velocities (e.g. see figure 8: *υ_{c1t1}* and *υ_{c1t2}*) can be calculated that can be used to select or discard parts of the video clip, or promote or demote certain parts accordingly as more or less relevant. The distance walked by cow 10-5 between figure 5A and 5B is clearly of higher velocity than between figure 5B and 5C, and is therefore of greater importance in the detection of locomotor system defects.

Figure 6 schematically illustrates a U-net type convolutional-deconvolutional neural network 100 that enables, when properly trained, to perform segmenting of the video sequence of images of the video clip in order to perform contour recognition of the body contours of animals. As an example, an image 40 from a video clip 56 is illustrated at the input of the machine learning data processing model 100. The U-net type convolutional-deconvolutional neural network 100 is known to be suitable for localized data labeling/classification. It enables not only to label/classify image features such as body contour 50 from an image 40, but also allows to determine the positions of these image features 50 in order to provide localization data. The exemplary U-net type machine learning data processing model 100 comprises a contracting path 102 having stages 105-1, 105-2 and 105-3 an expansive path 104 having stages 107-1, 107-2 and 107-3 and a bridging stage 106.

The contracting path 102 is typically configured as a conventional convolutional network with a sequence of layers wherein the original input image provided at a first spatial resolution is stepwise converted to a feature vector. A stage 105-1, 105-2 or 105-3 in the contracting path may for example comprise convolutional layers and a rectified linear unit (ReLU). An output of each stage 105-1, 105-2 and 105-3 may be provided to a corresponding stage 107-1, 107-2 and 107-3 in the expanding path 104 (not shown in figure 6, but typically represented by an arrow from stage 105-i to 107-i, where i=1, 2, .... N (n being the highest stage)). Also a down sampling module, e.g. a 2x2 max pooling operation with stride 2, is provided therein to provide for a downsampled mapping of that output to the next stage in the contracting path 102, so from 105-1 to 105-2, from 105-2 to 105-3, down to the lowest stage. This is indicated by the lines going between each two stages 105 respectively, in the contracting path 102. At each downsampling step the number of feature channels may be doubled.

The expansive path 104 performs a backwards conversion towards a representation at a higher resolution in the n-dimensional space, so as to provide localized labeling/classification information ŷfor the feature 50 in image 40. Each stage 107-1, 107-2 and 107-3 in the expansive path 104 may comprise an upsampling of the feature map followed by a convolution ("up-convolution") that halves the number of feature channels, a concatenation with the correspondingly cropped feature map from the contracting path 102, and further, convolutional layers followed by a ReLU. The upsampling together with the concatenation, will provide for the localization data to be preserved in the feature mask at the output. The bridging stage 106 may be provided to map each feature vector obtained from the final stage 105-3 in the contracting path 102 to the desired number of classes for labeling. The above type of machine learning data processing model 100, also referred to as a U-net type machine learning data processing model, is a known type of machine learning model and is for example described in Ronneberger, O., Fischer, P., Brox, T. (2015). U-Net: Convolutional Networks for Biomedical Image Segmentation. In: Navab, N., Hornegger, J., Wells, W., Frangi, A. (eds) Medical Image Computing and Computer-Assisted Intervention - MICCAI 2015. MICCAI 2015. Lecture Notes in Computer Science(), vol 9351. Springer, Cham. https://doi.org/10.1007/978-3-319-24574-4 28. The number of layers in the convolution path 102 and deconvolution path 104 (i.e. contracting path 102 and expansive path 104) is not limited to three but may be any number, dependent on the design. The output of the U-net type machine learning data processing model 100 typically ends with a 1x1 convolution in order to associate the feature data with the localization data. This will yield the body contour 50 of the cow 10.

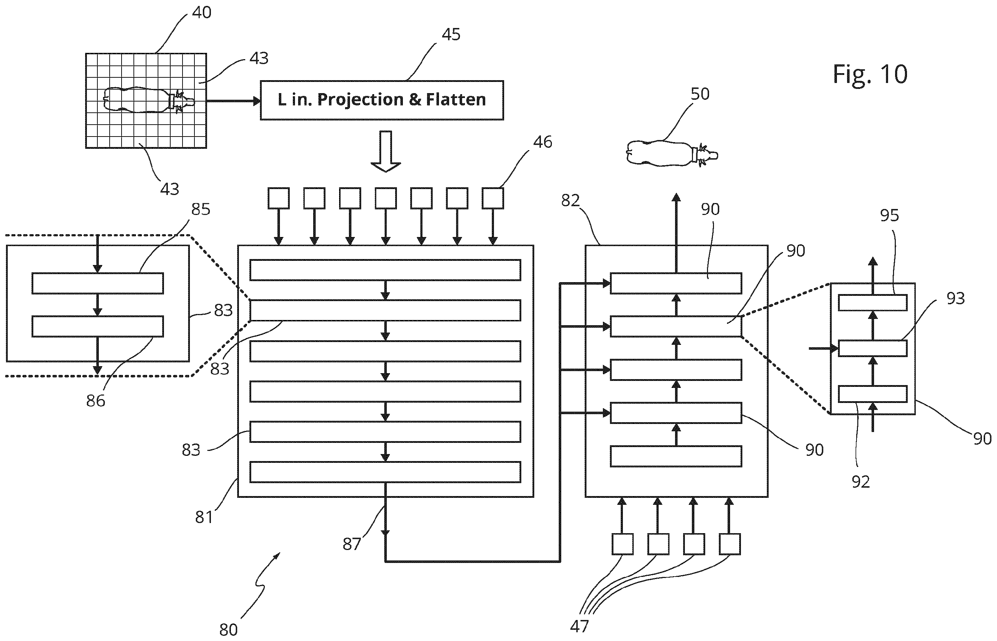

Another, alternative implementation that enables, when properly trained, to perform segmenting of the video sequence of images of the video clip in order to perform contour recognition of the body contours of animals, is the transformer 80 in figure 10. The transformer 80 comprises an encoder part 81 and a decoder part 82. Each one of the encoder 81 and decoder 82 comprises a plurality of encoder layers. The encoder layers 83 of encoder 81 each include a self-attention module 85 followed by a feed-forward module 86. The feed-forward module 86 comprises a multilayer perceptron (MLP), which transforms the output of the self-attention module 85 into an input for the next layer of the transformer 80. In the final encoder layer 83, the feed-forward module 86 provides the final output of the encoder 81. This output of the encoder 81 is provided as input to each decoder layer 90 in the decoder part 82.

At the input of the transformer 80, a linear projection and flattening module 45 breaks down the image 40 into patches 43, and converts the patches into embeddings 46 while associating each embedding with positional data. The embeddings 46 are provided as input to the first encoder layer 83 of encoder 81. Using the self-attention module in each encoder layer 83, the encoder 81 is able to relate the different features of image 40 to each other to thereby convey the not only the features themselves, but also their context in relation to other features of the image 40. This data is eventually provided, via the output 87 of the encoder, as input data to each decoder layer 90. The contextual data of the features of original image 40 is therefore preserved in the new image to be created by the decoder 82.

The decoder 82 starts with a plurality of default or blank output embeddings 47. Each of these embeddings 47 will be processed in each decoder layer 90 by going through a first self-attention module 92 to analyze the data in the input embedding 47 of that layer 90. Thereafter, in order to process the contextual and feature data from the original image 40, the second self-attention module 93 receives the output from the first self-attention module 92 and the encoder data 87, and after processing provides it to the feed-forward module 95, again a multilayer perceptron, in order to provide the output of the decoder layer 90. The output from the last decoder layer 90 will then provide the final output of the transformer 80, which is the body contour 50 of cow 10.

Figure 10 illustrates a regular vision transformer (ViT) architecture. However, a main advantage of the transformer architectures in general is that they are able to process multiple streams of related data in order to provide an orchestrated response. In the present context, for example, this may well be used in order to include temporal data from the video clips as well. As briefly explained already, including the temporal data for example makes it relatively easy to distinguish between different body contours 50 in the event that they are overlapping in an image (i.e. because the animals 10 are pushing each other or are standing contiguous to each other). As may be appreciated, any such situation is typically preceded by a situation wherein the cows 10 were separate from each other, so the information from individual body contours 50 of two different cows 10 is available in preceding images. This can be used to distinguish the different contours 50 in the image where they are overlapping, and hence enable motion analysis for individual animals 10 where these animal are too close or contiguous while walking. The analysis of temporal data in such video clips may be analyzed along with the other data from the images by using a video vision transformer (ViVit) for example. Although the architecture then becomes a bit more elaborate, the principle remains more or less the same.

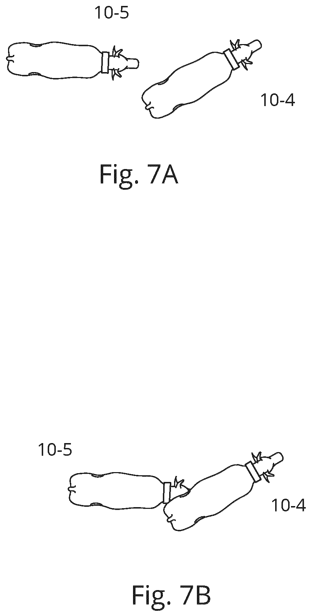

Back to figure 7A and 7B the situation of contiguous animals 10-4 and 10-5 is illustrated. As can be seen in figure 7B, it would be impossible to distinguish between the different body contours if only figure 7B would be considered, but using the temporal image data (i.e. here figure 7A) the body contours can be separated. In some implementations of the invention, use is made of bounding boxes in order to identify the whereabouts of an animal in an image, and in order to determine its speed. This may be combined with Kalman prediction from a tracker applied on the bounding boxes in order to separate and distinguish the body contours for two or more animals. In other implementations, the distinguishing between different contours is achieved using the further machine learning data processing models that perform the contour recognition. Various implementations are possible.

The method of the present invention is further illustrated in figure 8 in accordance with an embodiment. In figure 8, it can be seen that the video sequence consisting om images 40 at timestamps 52 corresponding to times *t₀, t₁, t₂,* ...... *tₙ₋₂, tₙ₋₁, tₙ,* is processed in order to obtain a video clip 56 thereof. The video clip 56 is provided to machine learning data processing model 60 in order to classify the motion to determine a locomotor score or classifier and provide that as classifier data 62. The classifier data may be a probability value, a class e.g., indicative of a range or a particular locomotor defect type for example, or a Boolean such as yes/no or '0' / '1'. However, from the clip 56, also the velocities of the animal 10 e.g. from frame to frame or across multiple frames or the whole clip is determined as velocity data 64. This velocity data 64 is used in order to evaluate the classifier data 62 in step 65. This has been described herein above in various examples. The result of the classification is then provided at the output 66.

An alternative implementation of the method of the invention, in accordance with another embodiment, is illustrated in figure 9. Here, the original video sequence has been split in several video clips 56 which may or may not be partly overlapping (both is allowed). The clips are designated clip 1, clip 2, clip 3 and clip 4. The clips 56 are provided as input to a machine learning data processing model 60 in order to classify the motion of the animal 10 in each video clip as being indicative of an abnormality in the locomotor system of the animal 10. Again the classification data 62 of each clip may be a probability value, a class e.g., indicative of a range or a particular locomotor defect type for example, or a Boolean such as yes/no or '0' / '1'. Parallel to this process, the video clips 56 are analyzed in order to determine the velocities characteristic for each clip 56, which is done in step 64. These velocities are provided to the input of module 63 which calculates weighing factors on the basis of the velocities. As may be appreciated, the weighing factors overall must be normalized, meaning that the sum of all weighing factors must add up to one (unity, 1). The calculated weighing factors 70 are illustrated as *w₂, w₂, w₃* and *w₄.* Dependent on the velocities 64, the classification data 62 of each clip 56 is weighed promoting the classification data 62 based on clips 56 with a higher velocity over the classification data 62 of the clips with lower velocity, in terms of relevance. Based on the weighing, the output is determined in step 75 and the result 76 provided as evaluation result by processor 4.

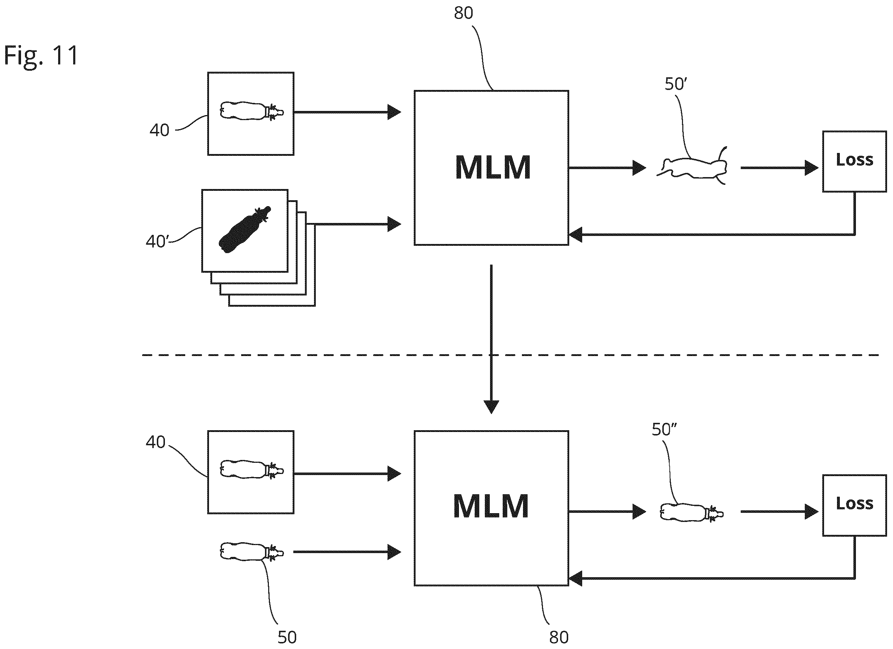

The present document describes several machine learning models 60, 80 and 100 in various contexts, for performing classification of motion from video clips to determine the presence or probability of an abnormality or defect in the locomotor system of an animal, but also for contour recognition and the separation of body contours 50 where they are overlapping in an image 40. Any of these machine learning data processing models may be trained using various different training strategies of either reinforced learning, supervised learning, unsupervised learning or combinations thereof, such as transfer learning strategies. For the classification of video clips to determine the presence or probability of an abnormality or defect in the locomotor system of an animal, an unsupervised learning strategy during pre-training of the models may be based on providing a large amount of video clips as training data, of arbitrary animals of the phenotype to be monitored (e.g. all cows, all sheep, all pigs, all horses, all cats, all dogs, all fish, all chicken etc.). If this is done on large amounts of data, the unsupervised learning process (an example of which will be described in relation to figure 11) will result in the machine learning data processing model to first start distinguishing normal motion of the animals from divergent motion. Later on, the machine learning data processing model will start to distinguish between different frequently occurring sorts of divergent motion, and hence start recognizing different sorts of abnormalities in the locomotor system of these animals. This may then be followed by fine-tuning, by applying a supervised learning strategy based on only small amounts clips to greatly improve the classification and even enable labelling thereof, i.e. designating the type of abnormality.

Similar, in order to train a machine learning data processing model 80 or 100 to perform contour recognition, the model may first be trained on large amounts of images of contours from animals of the phenotype to be monitored (e.g. all cows, all sheep, all pigs, all horses, all cats, all dogs, all fish, all chicken, or other moving subjects of a same type etc.) in an unsupervised learning approach during pre-training a pretext model. The contours in the images may be compared to slightly modified versions of these contours in terms of color, shape, texture, size, orientation, etc. The machine learning data processing model 80 or 100 will provide an outcome, which is compared with the modified versions in order to determine a loss using a cost function. The machine learning data processing model is then updated using backpropagation. Once pre-trained, the machine learning data processing model training is fine-tuned in a supervised learning process based on a small amount of data.

Figure 11 illustrates the training process schematically for the transformer model 80, however this training concept can be applied to any of the other described models as well. The upper part of the figure illustrates pre-training. Images 40 are provided to the machine learning data processing model 80 which establishes (or attempts to establish) the contour data 50' (which as illustrated is far from perfect). Based on the alternative images 40' which show slightly modified versions of the contour 50 in terms of color, shape, texture, size, orientation, etc., the loss will be calculated using a cost function. In fact it is established how far off the guestimate 50' is compared to the alternatives, and the parameters of the machine learning data processing model 80 are updated based thereon. This is continued until the pre-training results are good enough to implement the model and perform fine-tuning, e.g. on-site. In this next phase, in the lower half of the figure below the dashed line, the machine learning data processing model 80 is fed with images 40 from a camera 5, and ground truth data is provided illustrated as the real body contour 50. The machine learning data processing model 80 again estimates the body contour 50" which is already quite accurate, and the cost function thereafter calculates the loss based on the ground truth data 50. Again, the machine learning data processing model 80 is updated by modifying its parameters using backpropagation. The quality of the results are thereby greatly improved after implementation, based on only a very small data set.

The present invention has been described in terms of some specific embodiments thereof. It will be appreciated that the embodiments shown in the drawings and described herein are intended for illustrated purposes only and are not by any manner or means intended to be restrictive on the invention. It is believed that the operation and construction of the present invention will be apparent from the foregoing description and drawings appended thereto. It will be clear to the skilled person that the invention is not limited to any embodiment herein described and that modifications are possible which should be considered within the scope of the appended claims. Also kinematic inversions are considered inherently disclosed and to be within the scope of the invention. Moreover, any of the components and elements of the various embodiments disclosed may be combined or may be incorporated in other embodiments where considered necessary, desired or preferred, without departing from the scope of the invention as defined in the claims.

In the claims, any reference signs shall not be construed as limiting the claim. The term 'comprising' and 'including' when used in this description or the appended claims should not be construed in an exclusive or exhaustive sense but rather in an inclusive sense. Thus the expression 'comprising' as used herein does not exclude the presence of other elements or steps in addition to those listed in any claim. Expressions such as "consisting of", when used in this description or the appended claims, should be construed not as an exhaustive enumeration but rather in an inclusive sense of "at least consisting of". Furthermore, the words 'a' and 'an' shall not be construed as limited to 'only one', but instead are used to mean 'at least one', and do not exclude a plurality. Features that are not specifically or explicitly described or claimed may be additionally included in the structure of the invention within its scope. Any of the claimed or disclosed devices or portions thereof may be combined together or separated into further portions unless specifically stated otherwise, without departing from the claimed invention. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. Additions, deletions, and modifications within the purview of the skilled person may generally be made without departing from the spirit and scope of the invention, as is determined by the claims. The invention may be practiced otherwise then as specifically described herein, and is only limited by the appended claims.

## Claims

1. Method of monitoring a locomotor system of an animal by means of motion analysis, using image processing in a motion analysis system, the motion analysis system comprising a camera configured for monitoring at least a part of a pathway wherein the animal travels, wherein the camera is operatively connected to a computing device and a data storage of the analysis system, and wherein the computing device comprises a processor and wherein the computing device is communicatively connected to the data storage, the method comprising the steps of:
receiving, by the processor from the camera, a sequence of images of the animal moving along the pathway the sequence of images forming a video of the moving animal, wherein each image of the sequence of images is associated with a time stamp comprising timing data indicative of a moment of capturing the image by the camera;
detecting, by the processor, in a plurality of the images of the sequence the animal by recognizing in each image of the plurality of images a body of the animal;
selecting, by the processor, a subset of consecutive images from the sequence of images such as to form a video clip comprising the subset of consecutive images, wherein the video clip is representative of a fragment of the video;
determine, by the processor, using the time stamps of two or more images from the subset of consecutive images, a velocity of the animal;
classifying, by the processor using a machine learning data processing model, a motion of the animal from the video clip as being indicative of an abnormality in the locomotor system, and providing an outcome of said step of classifying as classification data at an output of the machine learning data processing model; and
providing, by the processor as an outcome of the method, an output signal dependent on the classification data being indicative of the abnormality in the locomotor system in the animal, wherein the output signal is further dependent on the velocity of the animal.

2. Method according to claim 1, wherein the step of classifying comprises at least one of:
determining, by the processor using the machine learning data processing model, whether or not the abnormality in the locomotor system occurs, wherein the classification data is a Boolean classifier indicative of the outcome of the classification; or
determining by the processor using the machine learning data processing model, a probability value indicative of a probability of the abnormality in the locomotor system occurring in the animal; or
determining by the processor using the machine learning data processing model, a classifier value indicative of an outcome within a range of outcomes, such as a class of abnormality or a range of probabilities.

3. Method according to claim 1 or 2, wherein the step of providing the output signal comprises a step of evaluating the classification data based on the velocity of the animal.

4. Method according to claim 3, wherein the step of evaluating comprises at least one of:
selecting or discarding the classification data based on the velocity of the animal being above or below a predetermined threshold, wherein the classification data is selected if the velocity is above the threshold, and wherein the classification data is discarded if the velocity is below the threshold; or
where the classification data comprises a probability value indicative of a probability of the abnormality in the locomotor system occurring in the animal, scaling the probability value based on the velocity, by weighing or multiplying the probability value with a weighing value which is dependent on the velocity.

5. Method according to any one or more of the preceding claims, further comprising the steps of:
receiving, by the processor from a radio frequency identification transceiver, an identification signal comprising identification data; and
associating the identification data with the animal.

6. Method according to any one or more of the preceding claims, wherein the step of selecting, by the processor, a subset of consecutive images from the sequence of images comprises
selecting, by the processor from the sequence of images, a plurality of subsets of consecutive images such as to form a plurality of video clips, each video clip of the plurality of video clips comprising a unique one of the subsets of consecutive images, wherein each video clip is representative of a unique fragment of the video, wherein optionally the fragments of two or more video clips at least partially overlap in time.

7. Method according to claim 6, wherein the step of selecting the plurality of subsets of consecutive images to form the plurality of video clips comprises:
applying a sliding window of a subset of consecutive images to the video, wherein for applying the sliding window, the step of selecting comprises the sub-steps of:
selecting, for each image of the sequence of images, a fixed number of consecutive images that precede the image concerned, wherein the fixed number determines a duration of the sliding window;
forming a set of images by arranging the consecutive images including the concerned image in sequential order in time, the set of images forming a momentary video clip of the sliding window, wherein the momentary video clip is associated with the image concerned;
associating the momentary video clip with the time stamp associated with the image concerned; and
forming the sliding window by providing each momentary video clip with the respective time stamp associated therewith, such as to yield the plurality of video clips.

8. Method according to claim 6 or 7, wherein the steps of classifying the motion of the animal and determining the velocity of the animal are performed for each video clip of the plurality of video clips for obtaining the classification data and velocity for each video clip, wherein the method further comprises:
determining, for each video clip and based on the velocity associated with the video clip, a weighing factor, wherein the weighing factor is dependent on and positively correlated with the velocity, and associating the weighing factor with the classification data associated with the video clip for indicating a significance thereof for each video clip; and
determining, for providing the output signal dependent on the classification data being indicative of the abnormality in the locomotor system in the animal, the output signal dependent on the classification data and the associated weighing factors of the plurality of video clips.

9. Method according to claim 8, wherein at least one of:
the weighing factor, for each video clip, is linearly dependent on the velocity;
the weighing factor, for each video clip, is clipped to either one of zero or unity dependent on whether the velocity is respectively below or above a threshold;
the weighing factor, for each video clip, is non-linearly dependent on the velocity.

10. Method according to any one or more of the claims 1-2 or 5-7, wherein the machine learning data processing model has been trained to provide at its output a probability value indicative of a probability of the abnormality in the locomotor system occurring in the animal based on receiving at its input the video clip and said velocity of the animal associated with the video clip.

11. Method according to any one or more of the preceding claims, wherein the output signal comprises at least one of: an indication of a grade of severeness of the abnormality in the locomotor system occurring in the animal, or a type of abnormality in the locomotor system occurring in the animal.

12. Method according to any one or more of the preceding claims, further comprising at least one step of:
controlling, by the processor dependent on the output signal, a separation gate for enabling separation of the at least one animal for further examination, such as a health check; or
provide the output signal to a display device, a mobile phone or a laptop, for presenting the information to an operator; or
store, based on the output data, an outcome of the method as animal management data associated with the animal in an animal management system.

13. Method according to any one or more of the preceding claims, wherein the step of detecting, by the processor in a plurality of the images of the sequence, the animal, comprises a step of segmenting the images such as to recognize a body contour of the animal.

14. Method according to claim 13, wherein the step of segmenting is performed by an image recognition module comprising an image recognition machine learning data processing model, wherein the image recognition machine learning data processing model has been trained to recognize a contour of an individual animal.

15. Method according to claim 14, wherein the image recognition machine learning data processing model has been further trained to separate two or more contours of individual animals, in a situation wherein the two individual animals are contiguous to each other such that their body contours blend together in the image.

16. Method according to any one or more of the preceding claims, wherein the camera is positioned above the pathway, such as to obtain image of the animal from above.

17. Method according to claim 16, wherein the step of detecting the animal further comprises a step of body feature recognition of the body of the animal, for recognizing at least one of a hip, a spine, a shoulder, a leg, a neck or a head of the animal.

18. Method according to any one or more of the preceding claims, wherein the machine learning data processing model, or the further machine learning data processing model is at least one of a group comprising: a neural network, such as a hierarchical neural network, a convolutional neural network, a convolutional-deconvolutional neural network, such as a U-net type neural network, a random forest model, a recurrent neural network, a long short-term memory, a vision transformer or a video vision transformer.

19. Method according to any one or more of the preceding claims, wherein at least one of:
the pathway is a lane or passage wherein the animal is enabled to move through; or
the pathway is provided by an area wherein the animal is enabled to move freely in multiple directions or a myriad of directions, the camera being configured to monitor the area, and wherein the method further comprises obtaining, from the each image of the subset of images of the video clip, an orientation of the animal and a position of the animal in the area, and segmenting the images of the video clip such as to correct a current orientation of the animal to a reference orientation of the animal, the reference orientation being predetermined, and wherein the step of determining the velocity of the animal further comprises determining the velocity along a trajectory following the positions of the animal obtained from the images of the subset.

20. Motion analysis system for detecting an abnormality in a locomotor system of an animal by means of motion analysis, the system comprising a camera configured for monitoring at least a part of a pathway wherein the animal travels, a computing device, and a data storage communicatively connected to the computing device, wherein the camera is operatively connected to the computing device and the data storage, and wherein the computing device comprises a processor, the processor being configured for controlling the system and for processing instructions which, when carried out, operate the system such as to perform a method comprising the steps of:
receiving, by the processor from the camera, a sequence of images of the animal moving along the pathway the sequence of images forming a video of the moving animal, wherein each image of the sequence of images is associated with a time stamp comprising timing data indicative of a moment of capturing the image by the camera;
detecting, by the processor in a plurality of the images of the sequence, the animal by recognizing in each image of the plurality of images a body of the animal;
selecting, by the processor, a subset of consecutive images from the sequence of images such as to form a video clip comprising the subset of consecutive images, wherein the video clip is representative of a fragment of the video;
providing, by the processor, the video clip as input to a machine learning data processing model, wherein the machine learning data processing model is trained to classify a motion of the animal from the video clip as being indicative of the abnormality in the locomotor system, and providing at an output of the machine learning data processing model an outcome of said classification as classification data;
determine, by the processor, using the time stamps of two or more images from the subset of consecutive images, a velocity of the animal; and
providing, by the processor as an outcome of the method, an output signal indicative of an occurrence of the abnormality in the locomotor system in the animal, wherein the output signal is based on the classification data and on the velocity of the animal.

21. System according to claim 20, wherein the system further comprises one or more radio frequency identification reader stations for communicating with one or more radio frequency identification transceivers, wherein the processor is further configured for processing instructions that enable the system to perform the steps of:
receiving, by the processor from at least one of the radio frequency identification transceivers, an identification signal comprising identification data; and
associating, by the processor, the identification data with the animal.

22. System according to claim 20 or 21, wherein the camera is positioned above the pathway, such as to obtain image of the animal from above.

23. System according to at least one of the claims 20-22, wherein the system further comprises a separation gate, the separation gate comprising actuation means that are operable by a control signal for operating the separation gate, wherein the processor is further configured providing a control signal for controlling, dependent on the output signal, the separation gate for enabling separation of the at least one animal for further examination, such as a health check.
